# EUROPEAN PATENT APPLICATION

(11) **EP 0 754 700 A2**
(43) Date of publication of application: **22.01.1997**
(21) Application number: 96304499.5
(22) Date of filing: 17.06.1996
(51) Int. Cl.: C07H 21/00, C07H 19/04, C12Q 1/68

(54) **Labelling and detection of nucleic acids**

(30) Priority: 17.06.1995 GB 9512380
(71) Applicant: Cruachem Limited, Glasgow G20 0UA (GB)
(72) Inventor: Davison, Amanda Ruth, Glasgow, G73 3SA (GB); Duckworth Gordon Stuart, Glasgow, G73 3SA (GB); Rao Vaman, Glasgow, G61 4HD (GB); Brown Thomas, Edinburgh EH10 6UR (GB); McClean John Paul, Edinburgh (GB)
(74) Representative: Ouzman, Beverley Nicola Claire

(57) **Abstract**

The invention described relates to 2,4-dinitrophenyl (DNP) labelling of nucleic acids or nucleic acid probes. Specifically, there is described compounds of formula I.

A compound of formula I:

X-Q-R*-NH-DNP (I)

wherein
DNP is a 2,4-dinitrophenyl group;
R* is a C₁₋₁₂ carbon chain, optionally interrupted with one or more of the following: -0-, -NH-, alkylamine, carbon-carbon double bond, carbon-carbon triple bond, carbonyl group or amide group;
Q is a group Q* or a group 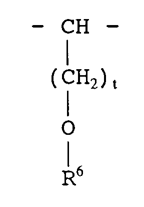
wherein t is O or is an integer of from 1 to 6;
Q* is a nucleoside comprising a heterocyclic amine base bonded to C1' of a C₅ or C₆ pentose sugar;
X is a group 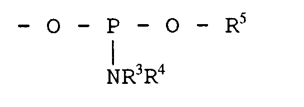 and where group Q represents a group Q*, X may also be a group 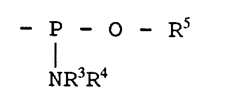
or a phosphate, diphosphate or triphosphate group;
R³ and R⁴ are each independently selected from C₁₋₆ alkyl, cycloalkyl, aralkyl and aryl groups; and
R⁵ and R⁶ are each blocking groups which may be the same or different
and the use of such compounds in the synthesis of oligonucleotide probes and formation of DNP-labelled oligonucleotides for use in solid-phase PCR, solid phase in situ hybridisation or in affinity chromatography.

## Description

The present invention relates to 2,4-dinitrophenyl (DNP) labelling of nucleic acids or nucleic acid probes and their use in a variety of applications in biomedical and genetic engineering fields.

DNP labelling of nucleic acids have very wide ranging applications which includes detection, sequencing and localisation of nucleic acid sequences of specific cells, cell extracts, tissue sections, chromosomes, etc. Specific applications include clinical diagnosis of nucleic acid containing pathogens, such as bacteria, viruses, fungi and diagnosis of genetic disorders.

Labelled DNA/RNA probes are widely used in a variety of diagnostic applications including the detection of pathogens by dot-blot analysis, *in situ* hybridisation gene sequencing and detection of PCR products (Diamandis E.P. et al, J Clin Lab Anal, 1993 7, 174-179; Igloi G.L. et al, BioTechniques, 1993, 15(3), 486-496; Mizukami et al, Biol Pharm Bull, 1993, 16(7), 729-731; Gibson K.M. et al, J. Virol Meth 1993, 43, 101-110). The use of non-radioactively labelled DNA/RNA probes rather than radioactively labelled probes is becoming increasingly popular although radioactive labelling is a well established method suited to most applications and is highly sensitive. However, radioactive labelling is hazardous, time consuming and expensive. In addition, radiolabelled oligonucleotides have a limited shelf life and can only be synthesised enzymatically on a very small scale. Non-radioactive labelling can overcome many of the disadvantages encountered with radioactive labelling. For example, non-radioactive labelling is safe, inexpensive, the labels have a long shelf life, no special training or disposal is required and non-radioactively labelled probes are reusable. The main disadvantage of non-radioactive labelling is its poor sensitivity. A solution to this problem is provided by the attachment of multiple labels and this has facilitated the visualisation of oligonucleotide probes in quantities previously obtainable only by radiolabelling (Misiura, K. et al, Nucleic Acids Res., 1991, 18, 4345-4354; Kessler, C. et al, Biol Chem Hoppe-Seylor, 1990, 371, 917-927).

Two types of non-radioactive labelling methods are available:
(a) those which can be detected directly and
(b) those which can be detected indirectly using immunocytochemistry techniques.

Direct Detection is provided by groups which are fluorescent or chemiluminescent. Fluorescent labelling of oligonucleotides is routine (Urdea, M.S. et al, Nucleic Acids Res, 1988, 16, 4937-4956; Schubert, F. et al, Nucleic Acids Res, 1990, 18, 3427; Smith, L.M. et al, Nature, 1990, 18, 5419-5423) but detection of oligonucleotides labelled in this way requires specialised equipment. Oligonucleotides can be conjugated to reporter groups, for example an enzyme such as horseradish peroxidase or alkaline phosphatase, which can be detected using colorimetric or chemiluminesence techniques. However synthesis and purification of these conjugates is lengthy (Urdea, M.S. et al, Nucleic Acids Res, 1988, 16, 4937-4956) and complicated and such conjugates are unsuitable for use in techniques such as PCR.

Indirect detection is used for reporter groups such as biotin which can be detected using enzyme linked immunosorbent assay (ELISA) methodology, with colorimetric or chomiluminescent visualisation via avidin or streptavidin conjugated to reporter enzymes such as alkaline phosphatase or horseradish peroxidase. Biotin is a widely used non-radioactive label and has been incorporated into oligonucleotides enzymically (Leary, J.J. et al, Proc Natl Acad Sci USA, 1983, 80, 4045-4049; Langer, P.R. et al, Proc Natl Acad Sci USA, 1981, 78, 6633-6637), photochemically (Forster, A.C, et al, Nucleic Acids Res, 1985, 13, 745-761), by reaction with amino-functionalised oligonucleotides (Cook, A.F. et al, Nucleic Acids Res, 1988, 18, 4077-4095; Viscidi, R.P. et al, Journal of Clinical Microbiology, 1986, 23, 311-317; Wachter, L. et al, Nucleic Acids Res, 1986, 14, 6227-6245), and by incorporation of biotinylated phosphoramidites during solid-phase synthesis (Misiura, K. et al, Nucleic Acids Res, 1990, 18, 4243-4354; Alves, A.M. et al, Tetrahedron Lett, 1989, 30, 3089-3092; Roget, A. et al, Nucleic Acids Res, 1989, 17, 7643-7651; Cocuzza, A.J., Tetrahedron Lett, 1989, 30, 6287-6290; Pon, R.T., Tetrahedron Lett, 1991, 32, 1715-1718). The main drawbacks are that biotin and its derivatives are expensive, the synthesis of biotinylated phosphoramidites is made difficult by the poor solubility of biotin, and endogenous biotin occurs at high levels in certain tissues, making it unsuitable for some types of *in situ* hybridisation.

The hapten digoxigenin (Kessler, C. et al, Biol Chem Hoppe-Seyler, 1990, 371, 917-927; Holtke, H.-J. et al, Biol Chem Hoppe-Seyler, 1990, 371, 929-939; Seibl, R. et al, Biol Chem Hoppe-Seyler, 1990, 371, 939-951; Muhlegger, K. et al, Biol Chem Hoppe-Seyler, 1990, 371, 953-965) is also detected indirectly. The labelled oligonucleotides are detected by digoxigenin-specific polyclonal sheep antibodies conjugated to alkaline phosphatase. Labelling with digoxigenin involves the enzymic incorporation of digoxigenin-labelled deoxyuridine-triphosphate into DNA, or via the reaction of a digoxigenin active ester with 5', 3' or internally-amino modified oligonucleotides. However the chemical labelling procedure is expensive and allows only the introduction of a single label whereas the enzymic method does not allow the controlled insertion of a specific number of labels.

In response to the need for an alternative non-radioactive label which can be used independently or in conjunction with other available chemical labels we have developed a system based on the 2,4-dinitrophenyl (DNP) group. DNP is a cheap and chemically simple hapten which is not found *in vivo*, and for which antibodies are readily available. The DNP group has been introduced into oligonucleotides via the action of deoxynucleotidyl terminal transferase or DNA polymerase on a DNP-aminohexyl derivative of ATP, and by the reaction of Sanger's reagent (2,4-dinitrofluorobenzene) with oligonucleotides containing an aminohexyl derivative of adenosine (Vincent, C. et al, Nucleic Acids Res, 1982, 10, 6787-6786). DNP groups have been introduced photochemically (Keller, G.H. et al, Anal Biochem, 1989, 177, 392-395) and by reaction with brominated bases (Keller, G.H. et al, Anal Biochem, 1988, 170, 441-450). Both single and multiple DNP groups have been incorporated into oligonucleotides using DNP phosphoramidites during solid phase synthesis, although in both examples of multiple addition the DNP group was unstable in aqueous ammonia under normal base-catalysed deprotection conditions (Roget, A. et al, Nucleic Acids Res, 1989, 17, 7643-7651; Will, D.W., et al, Carbohydrate Res, 1991, 216, 315-322).

The use of a DNP phosphoramidite derivative was reported by Brown et al, in Nucleic Acids Research, 21, No.8, p1705-1712 (1993). The phosphoramidite derivative was noted to be useful in labelling oligonucleotides by incorporation therein during solid phase synthesis. Oligonucleotides having single or multiple DNP-glycerol spacer labels were prepared and were proposed for use as probes in PCR products.

The present invention provides a range of novel compounds, in particular novel phosphoramidites which can be used in multiple DNP labelling of oligonucleotides, for example in solid-phase or solution phase synthesis, and novel nucleoside triphosphates which can be used to label nucleic acids following enzymic incorporation. The synthesis of the compounds and use of the compounds is also included as part of the invention, as described in more detail hereinafter.

The present invention provides compounds of formula I:

X-Q-R*-NH-DNP (I)

wherein
DNP is a 2,4-dinitrophenyl group;
R* is a C₁₋₁₂ carbon chain, optionally interrupted with one or more of the following: -O-, -NH-, alkylamine, carbon-carbon double bond, carbon-carbon triple bond, carbonyl group or amide group;
Q is a group Q* or a group 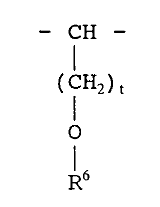
wherein t is 0 or is an integer of from 1 to 6, but preferably t is 1;
Q* is a nucleoside comprising a heterocyclic amine base bonded to C1' of a C₅ or C₆ pentose sugar. Optionally the nucleoside Q* may comprise one or more protecting groups R⁶;
X is a group 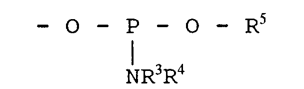
and where group Q represents a group Q*, X may also be a group 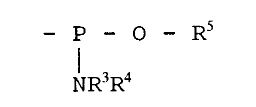
or a phosphate, diphosphate or triphosphate group;
R³ and R⁴ are each independently selected from C₁₋₆ alkyl, cycloalkyl, aralkyl and aryl groups; and
R⁵ and R⁶ are each blocking groups which may be the same or different.
R⁵ and R⁶ may be any suitable blocking groups known in the art. Mention may be made of the blocking groups 2-cyanoethyl and p-dimethoxytrityl.

In one particularly preferred embodiment group R⁶ is p-dimethoxytrityl and group R⁵ is 2-cyanoethyl.

Where group X is a group 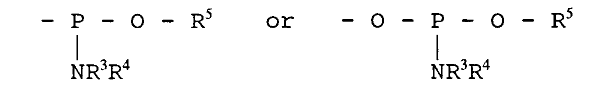 the compound of the invention is a phosphoramidite and may be used in the chemical synthesis (especially solid phase synthesis) of an oligonucleotide.

Where group X represents a phosphate group, the compound according to the present invention will be a nucleotide derivative or a diphosphate or triphosphate nucleoside derivative. A nucleoside triphosphate derivative according to the invention is particularly useful for enzymic incorporation to form a nucleotide chain, for example in PCR.

Group R* may optionally be a group (CH₂)ₙ-O-(CH₂)ₘ, -C=CR¹-, -C≡C- or -C-CR¹R², wherein R¹ and R² may each independently be a hydrogen atom, C₁₋₆ alkyl, a group -(CH₂)ₚNHC=O(CH₂)_{q}-, wherein n, m, p and q may each independently be 0 or an integer of from 1 to 12, especially 1 to 6.

In a preferred embodiment group R* may be a methylene, ethylene, n-propylene, n-butylene group, or a group -(CH₂)ᵣ-NHC(=O)-(CH₂)ₛ-, wherein r is 1, 2 or 3 and s is 3, 4, 5 or 6.

The heterocyclic amine base of group Q* may be, for example a purine, such as adenine, guanine or derivatives thereof, or a pyrimidine, such as cytosine, uracil, thymidine or derivatives thereof. As derivatives may be mentioned alkylated derivatives (especially methylated derivatives) and halogenated derivatives, but are not specially limited thereto. Uracil and derivatives thereof are specially preferred.

Examples include the compounds of the general formulae IIa-IId.
IIa where X is a mono-phosphate group: 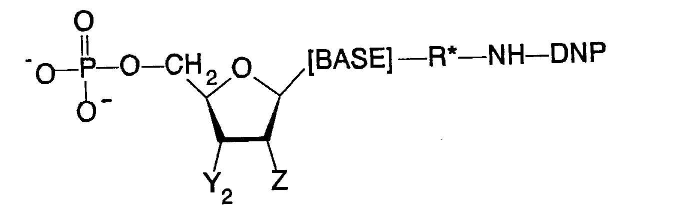
IIb where X is a di-phosphate group: 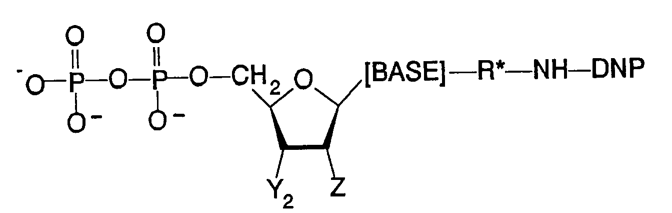
IIc where X is a tri-phosphate group: 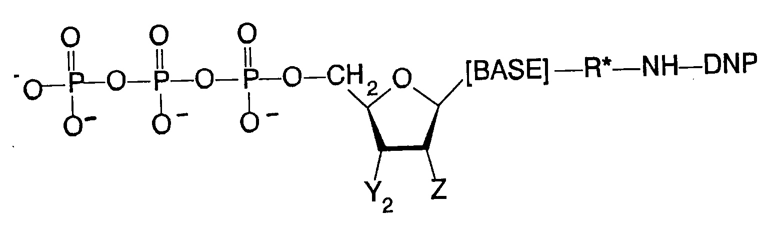
IId where X is a group

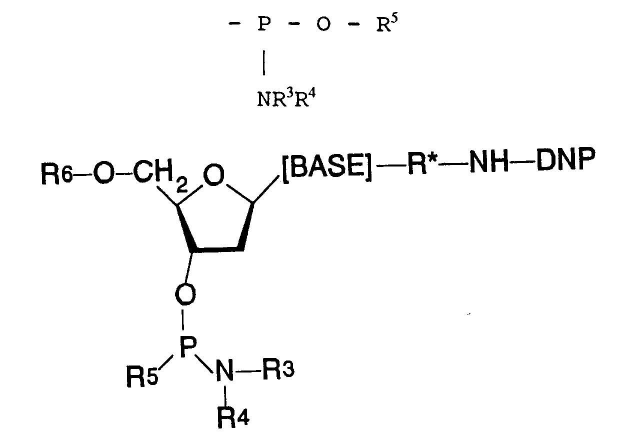

In the formulae IIa and IId, groups R*, R³, R⁴, R⁵ and R⁶ are as defined above for formula I;
[BASE] refers to a heterocyclic aime base (for example a pyrimidine or purine, or an alkylated derivative thereof, for example uracil and methylated uracil);
Group Y₂ is a hydrogen atom or a hydroxyl group; and group Z represents a hydrogen atom, or a group -OR⁷, -NHR⁷, -SR⁷, where R⁷ is a blocking group, a C₁₋₆ alkyl group or a hydrogen atom.

The C₅ to C₆ pentose sugar of group Q* is preferably of formula IIe: 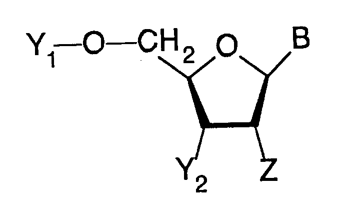 wherein:
one of groups Y₁ and Y₂ represents a group X and the other of groups Y₁ and Y₂ represents a hydrogen atom, or an hydroxyl, C₁₋₆ alkyl or -OR⁶ group;
group Z represents a hydrogen atom, or a group -OR⁷, -NHR⁷, -SR⁷ where R⁷ is a blocking group, a C₁₋₆ alkyl group or a hydrogen atom;
R⁶ is as defined above; and
B represents a covalent bond linking the pentose sugar moiety to the heterocyclic amine base of group Q*.

Desirably where group Y₁ is a group X, then group X will be a mono-, di- or tri-phosphate group. In this preferred embodiment group Y₂ advantageously may represent a hydrogen atom, or an hydroxyl group.

Desirably where group Y₂ is a group X, then group X will be a group 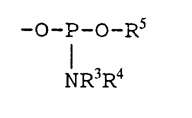 wherein groups R³, R⁴ and R⁵ are as defined above. In this preferred embodiment group Y₁ advantageously may represent a group -OR⁶ (where group R⁶ is as defined above).

Preferred pentose sugar moieties of formula IIe include 2-deoxyribose, 2,3-dideoxyribose, ribose 2'-O-alkylribose

Desirably where group Q is a nucleoside (i.e. is a group Q*), group R* is attached to the nucleotide base of Q*. Where the base is a pyrimidyl group, R* is located at position 5. Preferably the base is also attached to the sugar moiety of Q* at position 1 (as illustrated in formulae IIIa and IIIb below). Where the base of group Q* is a purine group, the base may be linked to the sugar moiety of Q* at position 3 and to group R* at position 8, or if the base group of Q* is a 7-deazapurine, the R* group may be attached at position 7.

Examples of the nucleoside based DNP phosphoramidites of the present invention include those of formulae IIIa and IIIb below:

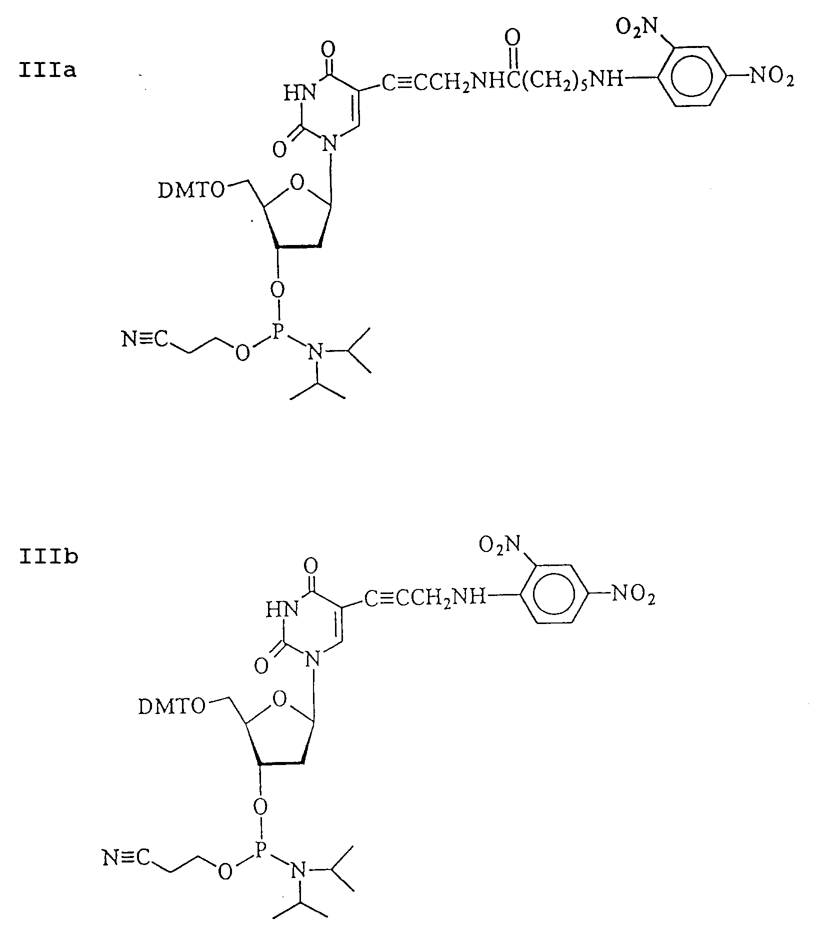

Preferred phosphoramidites have hydrogen bond donor-acceptor sites available for Watson-Crick base pairing. A triple bond situated α to C-5 ensures optimal hybridisation characteristics.

A suitable scheme for preparation of a phosphoramidite is set out below (details of this synthesis are given in Example 1):

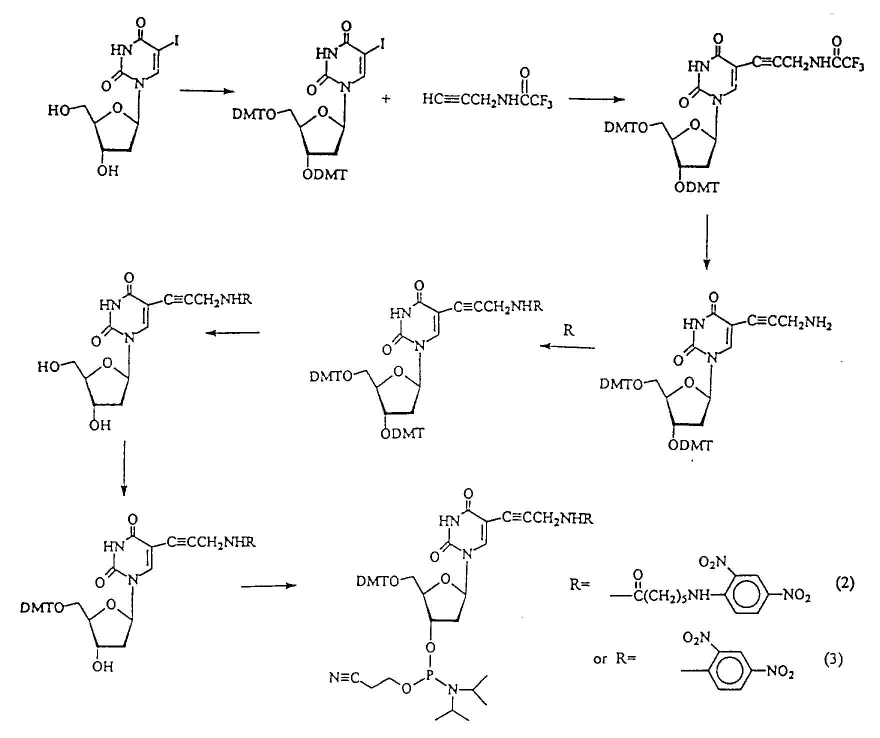

The present invention also provides the use of the abovementioned compounds to synthesise oligonucleotide probes. In this aspect, the phosphoramidites of the present invention are particularly suitable since the duplex melting temperatures enable binding to be highly selective.

In a further aspect the present invention provides DNP labelled oligonucleotides formed using a compound of the present invention (normally a phosphoramidite or nucleoside triphosphate derivative). The use of the DNP labelled oligonucleotides as probes is also included in this invention. The probes may also be useful in solid phase PCR, in solid phase in situ hybridisation and in affinity chromatography.

Thus, for example, an anti-DNP antibody may be localised on a support (for example be covalently bonded to beads, membrane, rods and the like). Introduction of a DNP-labelled oligonucleotide or compound according to the invention would become bound in a specific manner to the antibody, enabling separation of the DNP labelled moiety from any contaminants. After washing to remove any unbound matter the DNP labelled moiety or the DNP labelled moiety/antibody complex could if desired by released, for example by use of a suitable medium, or may be further reacted, in situ.

Thus, DNP labelled oligonucleotides could be specifically retained by an anti-DNP antibody and then subjected to PCR.

The present invention further comprises a process for labelling a hydroxyl group containing chemical moiety (usually with a DNP-label), said process comprising providing a compound of formula I above and contacting said compound with said hydroxyl group containing chemical moiety (such as an hydroxyalkyl, hydroxyaralkyl, amino acid, peptide, hydroxycycloalkyl, carbohydrate, nucleoside, nucleotide or oligonucleotide moieties).

Optionally said process comprises the further step of detecting said labelled moiety, wherein an anti-DNP agent, such as an anti-DNP antibody, may be used. Enzymic methods of detection may be employed as known in the art.

### Figure 1

Comparison of antibody mediated detection of glyceryl and deoxyruridine based DNP-labelled oligonucleotides 0.5 µl aliquots of these dilutions were applied to nylon membrane and developed with anti-DNP/HRP conjugate.

### oligonucleotide sequences

A = 5'-GGX GGC CAA CGA CGA GGC CGC GCX GCT GGA GTT GAT XGC C-3'
   where X = deoxyuridine based DNP label.
B = 5'-TYY YGG TGG CCA ACG ACG AGG CCG CGC TGC TGG AGT TGA TTG CG-3'
   where Y = glyceryl based DNP label.
C = 5'-ACG GGA TCA TCG ACG AAA TTG TTA ACG GGT-3'

Lane No 1 contained 16ng oligonucleotide; No 2 3.2ng; No 3 640pg; No 4 128pg; No 5 25pg; No 6 5pg; No 7 unlabelled control (oligonucleotide C).

### Figure 2

Comparision of antibody mediated detection of oligonucleotides containing DNP monomers with and without C6 linkers. 0.5 µl aliquots of the oligonucleotides at the dilutions indicated below were applied to a nylon membrane and developed with anti-DNP/HRP conjugate and DAB/H₂O₂.

### oligonucleotide sequences (5' to 3')

A = TCT CAA AGT CGT GAA LTA ACT TTT AAL ACC TTC ALA TCG
   where L=DNP DU monomer with C6 linker
B = TCT CAA AGT CGT GAA STA ACT TTT AAS ACC TTC ASA TCG
   where S=DNP DU monomer without C6 linker
C = TCT CAA AGT CGT GAA TTA ACT TTT AAT ACC TTC ATA TCG

Lane No 1 contained unlabelled control (oligonucleotide C); No 2 14ng of test oligonucleotide; No 3 2.8ng; No 4 560pg; No 5 112 pg; No 6 22pg; No 7 5pg; and No 8 water.

### Figure 3

The effect of using large numbers of DNP labels. A series of dilutions of the oligonucleotide pool were made and 0.5 µl aliquots of each dilution were applied to a nylon membrane. In this case the membrane was developed using an anti-DNP/alkaline phosphatase conjugate as described previously.

### oligonucleotide sequences (5' to 3')

TXn GGT TGA TCY GGA CAA TGA CGG YTA CGG AGG TGG TTG YGG C
   TXn GGY GGC CAA CGA CGA GGC CGC GCY GCT GGA GTT GAT YGC G
   TXn GCT TTC ATG YGG TTG CTG TGY TGG ATG GCC GAA GGA GAY T
   TXn GTG TCC TTA CAC AGC GAY CCA ATC GCA YCT CTC AAT YAG C
   TXn TTA ATA ACC AYG CAG TAA TGG TCG GCC YTA CCG GCG YCC A
Pool A
   X = glyceryl DNP;n = 3;Y = DNPdU
Pool B
   X = glyceryl DNP;n = 17;Y = DNPdU

Lane No 1 contains 16ng of oligonucleotides; No 2 8ng; No 3 4ng; No 4 1ng; No 5 200pg; No 6 40pg; No 7 8pg; No 8 1.6pg; No 9 320fg; No 10 64fg; No 11 non-DNP labelled oligonucleotide.

### Figure 4

Hybridisation of DNP-labelled oligonucleotides to immobilised target DNA

Serial dilutions of a double stranded target DNA (M.a. paratuberculosis) were heated to 90°C, snap-cooled and applied to a nylon membrane. The membrane was fixed, blocked and incubated for 1 hour at ambient temperature with a 1000 fold excess of a DNP labelled probe pool (5 oligonucleotides, each with 3 terminal and 3 internal DNP labels) then developed using the anti-DNP/alkaline phosphatase conjugate and BCIP/NBT.

### oligonucleotide sequences

C(+) 5'-GTT CGG GGC CGT CGC TTA GG
   C(-) 5'-CCC ACG TGA CCT CGC CTC CA
1(+) 5'-GTT CGG GGC CGT CGC TXA GG
   1(-) 5'-CCC ACG TGA CCT CGC CXC CA
3(+) 5'-GXT CGG GGC CGX CGC TXA GG
   3(-) 5'-CCC ACG XGA CCX CGC CXC CA

X=DNP C6 dU monomer.

Lane No 1 contained 7.5ng oligonucleotide; No 2 3.8ng; No 3 1.9ng; No 4 0.9ng; No 5 0.5ng; No 6 0.2ng and No 7 non-target DNA control.

### Figure 5

The effectiveness of PCR primers labelled internally with the dU DNP monomer (2)

### Figure 6

Gel analysis of PCR products incorporating DNPdUTP

In the PCR reactions varying ratios of DNPdUTP to TTP were used as follows: Lane 1) All DNPdUTP; 2) 10 DNPdUTP : 1TTP; 3) 5 DNPdUTP : 1 TTP; 4) 2 DNPdUTP : 1 TTP; 5) 1 DNPdUTP : 1 TTP; 6) 1 DNPdUTP : 2 TTP; 7) 1 DNPdUTP : 5 TTP 8) All TTP.

Each reaction tube contained 5µl 10 x PCR buffer, ATP, CTP and GTP (0.2mM each) and an equal volume of the relevant DNPdUTP/TTP mixture, Taq polymerase (2 Units), M.a. paratuberculosis template (5ng) and primers (1µM each) made up to 50µl with distilled water and overlaid with 100µl of mineral oil. The PCR cycle is listed in the experimental section. The reaction products were analysed by running a 1% agarose gel stained with ethidium bromide.

### Figure 7

Immunoblot comparison of products yielded from PCR experiment

In the lanes from left to right the following DNPdUTP:TTP ratios were used:

All DNPdUTP; 10/1; 5/1; 2/1; 1/1; 1/2; 1/5; and all TTP.

The invention may be further illustrated with reference to the following examples:

### Example 1

### Synthesis of C6 dU DNP phosphoramidite (1)

### (a) 3-N-trifluoroacetylamidoprop-l-yne (2)

To a chilled (0°C) solution of trifluoroethylacetate (55.35g, 0.39mol) in anhydrous methanol (225ml) was added propargylamine (16.5g, 0.3mol) dropwise with stirring. The reaction was stirred at ambient temperature for 3.5 hours. The reaction mixture was evaporated *in vacuo* to an oil, dissolved in dichloromethane (675ml) and washed with saturated NaHCO₃ (2 x 675ml) followed by saturated aqueous KCl (225ml). The organic portion was dried (Na₂SO₄), filtered and evaporated *in vacuo* to an orange oil (36.9g, 81% yield. IR data uₘₐₓ/cm⁻¹:3480-3070 (br, m, NH), 1730 (s, C=O).

### (b) 5'3'-O-Bisdimethoxytrityl-5-iodo-2'-deoxyuridine (3)

5-Iodo-2'-deoxyuridine (25g, 0.07lmol) was coevaporated with pyridine (250ml x 3) and dissolved in pyridine (250ml). To this solution was added a solution of 4,4'-dimethoxytrityl chloride (59.75g, 0.176mol) in anhydrous pyridine (120ml) and dichloromethane (80ml) dropwise at ambient temperature over 30 minutes and the mixture was stirred at ambient temperature overnight. The reaction was quenched by the addition of methanol (25ml), evaporated to a gum, dissolved in dichloromethane (1.01) and washed with saturated NaHCO₃ (2 x 300ml). The organic portion was dried (Na₂SO₄), filtered and evaporated *in vacuo*. The resulting oil was dissolved in dichloromethane (600ml) and precipitated into hexane (4.01). The precipitate was filtered, dissolved in dichloromethane, evaporated and dried *in vacuo* overnight to give the product as an off white foam 72.0g. The product was used without further purification.

¹H n.m.r. data (CDC1₃): δH 1.8-2.0(m, 2H, 2'H), 2.9-3.2(m, 2H, 5'H), 3.7(s, 12H, 4 x OCH₃), 3.9(s, 1H, 3'H), 4.38-4.4(m, 1H, 4'H), 6.32-6.36(m, 1H, 1'H), 6.71-6.82(m, 8H, ArH), 7.2-7.4(m, 18H, ArH), 8.1(s, 1H, NH), 9.4(s, 1H, C=CH).

### (c) 5-(3-(N-trifluoroacetylamido)prop-1-ynyl)-3',5'-O-bisdimethoxytrityl-2'-deoxyuridine (4).

To a solution of compound (3) (64.0g, 0.067mol) in dry degassed DMF (500ml) was added copper iodide (2.5g, 0.0134mol), dried and distilled triethylamine (33.0ml), 3-*N*-trifluoroacetylamidoprop-1-yne (1) (30.7g, 0.203mol) followed by tetrakistriphenylphosphine palladium (0) (7.8g, 0.0068mol) under argon. The resulting clear brown solution was stirred under argon at ambient temperature overnight. After which time the reaction was found to be complete. Methanol (500ml), dichloromethane (500ml) and Dowex-bicarbonate 1-X8 (37.0g) were added to the reaction mixture and left at ambient temperature for 1 hour with occastional swirling. This mixture was then filtered and the filtrate was evaporated *in vacuo* to give a brown oil. The oil was triturated with water (150ml) giving a solid which was dissolved in DCM (400ml), dried (Na₂SO₄), filtered and evaporated *in vacuo* to give a foam (95.9g), which was used without further purification.

### (d) 5-(3-(amino)prop-1-ynyl)-3',5'-O-bisdimethoxytrityl-2'-deoxyuridine (6)

To a solution of compound 4 (93.0g, 0.0947 mol) in dichloromethane (600ml) was added aqueous 2M NaOH (600ml). The two phase mixture was shaken periodically and followed by tlc (10% methanol/dichloromethane). When the reaction was complete (2 hours) the two layers were allowed to separate. The organic portion was dried (Na₂SO₄), filtered and evaporated *in vacuo* giving a brown foam (71.0g). The product was purified by wet-flash silica gel chromatography eluting with 1% methanol/dichloromethane to give the product as a pale brown foam (36g, 43%).

¹H n.m.r. data (CDC1₃): δH 1.14-1.18(m, 2H, CH₂), 1.8-2.0(m, 2H, 2'H), 2.7-2.85 (m, 2H, 5'H), 3.7(s, 12H, 4 x OCH₃), 3.9(s, 1H, 3'H), 4.39-4.41(m, 1H, 4'H), 6.40-6.43(m, 1H, 1'H), 6.73-6.79(m, 8H, ArH), 7.2-7.4(m, 18H, ArH), 7.65-7.80(br s, 1H, NH), 8.1(s, 1H, C=CH).

### (e) N-(2,4-dinitrophenyl)-6-aminohexanoic acid (5)

To a suspension of aminohexanoic acid (10.1g, 0.077mol) in anhydrous methanol (200ml) was added triethylamine (29.9ml) in one portion and dinitrofluorobenzene (9.65ml, 0.077mol) over 1 minute. The reaction mixture was stirred at ambient temperature overnight and then evaporated *in vacuo*. The resulting oil was recrystallised from anhydrous methanol (200ml), filtered, washed with cold ethanol and dried *in vacuo*. The filtrate was concentrated to about 50ml, dicholormethane (450ml) was added and washed with 10% citric acid (300ml) and water (300ml). The organic portion was dried (Na₂SO₄) and evaporated to a yellow solid which was recrystallised from ethanol, dried *in* *vacuo* and combined with the product obtained earlier (18.65g, 82%).

### (f) 5-(3-(2,4-dinitrophenyl hexanamido)prop-l-ynyl)-3',5'-O-bisdimethoxytrityl-2'-deoxyuridine [7]

To a solution of compound [5] (6.9g, 0.0232mol) and *N*-hydroxysuccinimide (3.3g) in DMF (80.0ml) was added a solution of DCC (7.0g, 0.0336mol) in DMF (17.0ml). The resulting mixture was stirred at ambient temperature overnight. The reaction mixture was filtered and the filtrate was added to a solution of compound [6] (15.0g, 0.016mol) in DMF (100ml). The resulting dark brown solution was stirred at ambient temperature and followed by tlc (7.5% methanol/dichloromethane). The reaction mixture was evaporated under high vacuum to give a brown oil which was dissolved in dichloromethane (200ml) and washed with water (250ml x 2). The organic layers were combined, dried (Na₂SO₄), concentrated *in vacuo* and precipitated in hexane (21). The precipitate was removed by filtration, dissolved in dichloromethane and evaporated *in vacuo* giving the product as a yellow foam (24.5g) which was used without further purification.

### (g) 5-3(-(2,4-dinitrophenyl hexamamido)prop-1-ynyl)-2-deoxyuridine [8]

To a solution of compund [7] (19.7g, 0.0169mol) in anhydrous dichloromethane (200ml) was added distilled pyrrole (17.6ml, 0.253mol) and 3% trichloroacetic acid in dichloromethane (480ml). The reaction mixture was stirred at ambient temperature and followed by tlc (10% methanol/dichloromethane). After 30 minutes the reaction was complete. The pH was adjusted to 7 by the addition of triethylamine, the mixture was evaporated and the resulting brown oil was dried *in vacuo* overnight. The product was purified by wet-flash silica gel chromatography eluting with 7% methanol/dichloromethane to give the product as a yellow foam (6.26g, 66%).

¹H n.m.r. data (CDC1₃); δH 1.28-1.39(m, 2H, CH₂), 1.46-1.68(m, 4H, 2 x CH₂), 2.05-2.18(m, 4H, CH₂, 2'H), 3.40-3.50(m, 2H, COCH₂) 3.55-3.67(m, 2H, 5'H), 4.05-4.10(m, 2H, NCH₂), 4.20-4.28(m, 1H, 3'H), 6.10-6.15(m, 1H, 4'H), 7.15-7.20(m, 1H, 1'H), 8.15(s, 1H, C=CH), 8.16-8.34(m, 2H, DNP H⁵, H⁶), 8.72-8.83(m, 2H, DNP H³,NH);

F.a.b. mass spectrum: m/z 561.19133 (M+H)⁺ calc. for C₂₄H₂₈N₆O₁₀, m/z 560.18669.

### (h) 5-(3-(2,4-dinitrophenyl hexanamido)prop-1-ynyl)-5'-O-dimethoxytrityl-2'-deoxyuridine [9]

Compound [8] (6.26g, 0.011mol) was coevaporated with pyridine (30ml x 3) and dissolved in pyridine (50ml). To this solucation was added dropwise a solution of 4,4'-dimethoxytrityl chloride (4.17g, 0.0123mol) in dichloromethane (30.0ml) and pyridine (3.0ml) with stirring at ambient temperature. The reaction mixture was stirred at ambient temperature for 3 hours after which time the reaction was found to be complete by tlc analysis (7.5% methanol/dichloromethane). The reaction was quenched by addition of methanol and the pH was adjusted to 7 by the addition of triethylamine. The mixture was evaporated *in vacuo* to an oil, coevaporated with dichloromethane/toluene twice and dried *in vacuo*. The product was purified by wet-flash silica gel chromatography eluting with 2% methanol/dichloromethane to give the product as a pale brown foam (7.0g, 7.3%).

¹H n.m.r. data (CDC1₃); δH 1.15-1.27(m, 2H, CH₂), 1.30-1.45(m, 2H, CH₂), 1.50-1.75(m, 4H, 2 x CH₂), 1.90-2.00(m, 2H, 2'H), 2.20-2.55(m, 2H, COCH₂), 2.80-2.93(m, 2H, 5'H), 3.25-3.40(m, 2H, NCH₂), 3.70(s, 6H, OCH₃), 4.10(s, 1H, 3'H), 4.50(s, 1H, 4'H), 5.95-6.00(m, 1H, 3'OH), 6.25-6.30(m, 1H, 1'H) 6.75-6.90(m, 5H, ArH), 7.10-7.30(m, 8H, ArH), 7.36-7.42(m, 1H, DNP H⁶), 8.10(s, 1H, C=CH), 8.15-8.20(m, 1H, DNP H⁵), 8.48-8.54(m, 1H, DNP NH), 9.00(s, 1H, DNP H³);

F.a.b. mass spectrum: m/z 863.32030 (M+H)⁺ calc. for C₄₅H₄₆N₆O₁₂, 862.31737.

### (i) 5-(3-(2,4-dinitrophenyl hexanamido)prop-1-ynyl)-5'-O-dimethoxytrityl-3'-(2-cyanoethyl-N,N-diisopropylphosphoramido)-2'-deoxyuridne [10]

To a solution of compound [8] (5.0g, 0.0058mol) in anhydrous dichloromethane (60.0ml) was added diisopropylammonium tetrazolide (0.41g, 0.0029mol) followed by 2-cyanoethyl N,N,N',N'-tetraisopropylphosphorodiamidite (1.78g, 0.0069mol). The reaction mixture was stirred at ambient temperature and monitored by hplc. The reaction was found to be complete after 4 hours. Dichloromethane (50.0ml) was added and the reaction mixture was washed with saturated NaHCO₃ (80.0ml x 2). The organic portion was dried (Na₂SO₄), filtered and evaporated *in vacuo* to a foam. The product was purified by wet-flash silica gel chromatogrphy eluting with 2% isopropanol/ dichloromethane to give the product as yellow power (5.46g, 89%).

¹H n.m.r. data (CDC1₃): δH 1.05-1.10(m, 2H, CH₂), 1.13-1.20(m, 12H, 4 x CH₃), 1.30-1.45(m, 2H, CH₂), 1.50-1.70(m, 4H, CH₂), 1.88-1.98(m, 2H, 2'H), 2.30-2.45(m, 2H, COCH₂), 2.50-2.65(m, 2H, 5'H), 3.25-3.68(m, 6H, NCH₂ 2 x CH₂), 3.75(s, 6H, OCH₃), 4.10-4.20(m, 1H, 3'H), 4.55-4.65(m, 1H, 4'H), 6.20-6.30(m, 1H, 1'H), 6.75-6.90(m, 6H, ArH), 7.15-7.35(m, 7H, ArH), 7.36-7.45(m, 1H, DNP H⁶), 8.13-8.22(m, 2H, DNP H⁵, C=CH), 8.45-8.55(m, 1H, DNP NH), 9.05(s, 1H, DNP H³);

³¹P n.m.r. data (CDC1₃); δP 148.76 (s) and 148.52 (s).

F.a.b. mass spectrum: m/z 1063.43657 (M+H)⁺ calc. for C₅₄H₆₃N₈N₁₃P, m/z 1062.42522.

### (j) 5-(3-(2,4-dinitrophenyl hexanamido)prop-1-ynyl)-2'-deoxyuridine-5'-triphosphate [11]

The dry DNP labelled nucleoside precursor [compound 8], (400 mg, 0.714mmol) was stirred at 0°C in dry trimethylphosphate (1ml) with powdered proton sponge (1,8-bis(dimethylamino)naphthalene, 230mg, 1.5eq). To this was added, dropwise, anhydrous phosphorus oxychloride (0.124ml, 1.1eq) with stirring at 0°C. After thin layer chromatography showed this stage of the reaction to be complete (nPrOH: NH₄OH: H₂O 11:7:2, product R₁=0.15), a mixture of 0.5M bis-tri-n-butylammonium pyrophosphate in anhydrous DMF (7.0ml, 5eq.) and n-tributylamine (0.70ml) was added with vigorous stirring at 0°C. After 1 minute 0.2M aqueous Et₃N.H₂CO₃(pH 7.0, 35ml) was added to the reaction mixture. All solvents were removed in vacuo and the crude product was purified by column chromatography on DEAE cellulose (Sigma) using a linear gradient of Et₃N.H₂CO₃ between 0.02 and 0.80M at ambient temperature. The 5'-triphosphate was collected and converted to the tetrasodium form using Dowex-50 resin. The tetrasodium DNP labelled triphosphate (DNPdUTP) was then lyophilized to give the product as a yellow foam (110mg, 23%).

³¹P NMR data (d₆-acetone) δP = -22.63 (t, J=19.9 Hz), -11.12 (d, J=20.2 Hz), -9.68 (d, J=19.5 Hz). E260mm (H₂O) = 9.375; Emax: 216nm (17,514) 279nm (12,175), 364nm (13,442);

F.a.b. mass spectrum : m/z 889 (m+H)⁺

### Example 2

### Comparison of detection sensitivity of DNP glycerol and DNP C6 dU labelled oligonucleotides

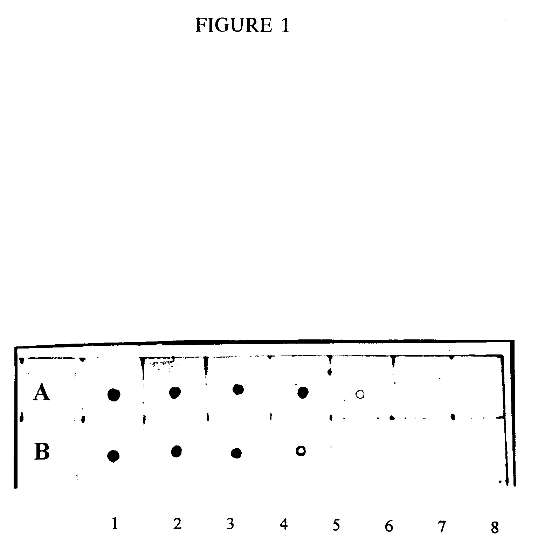

To compare the detection sensitivity of oligonucleotides labelled with the glyceryl-based DNP monomer and the deoxyuridine-based DNP monomer two oligonucleotides were synthesised. One was made with three DNP C6 dU residues (of formula II) distributed within the sequence and the second with three DNP glycerol monomers at the 5'-end. Decreasing amounts of each oligonucleotide were fixed to a nylon membrane and treated with an anti-DNP antibody-HRP conugate and DAB (figure 1). The results show that both types of DNP label give the same detection sensitivity and that it is possible to detect both DNP labelled oligonucleotides in amounts as low as 25pg.

### Example 3

### The effect of the hexamethylene linker in the DNP C6 dU monomer on detection sensitivity

An immunoblot experiment was carried out to determine whether or not the presence of the hexamethylene (C-6) linker in the DNP C6 dU monomer enhances the sensitivity of detection by an anti-DNP anitbody-HRP conjugate. A deoxyuridine based monomer (3) was synthesized which did not contain the C-6 linker (DNP dU) and three of these units were incorporated into an oligonucleotide. An oligonucleotide of the same sequence was also made with the DNP labels attached using the DNP C6 dU monomer.

All immunoblot experiments were carried out on Hybond-N 0.45 micron nylon membrane (Amersham) in phospate buffered saline (PBS) solution (prepared from tablets supplied by Sigma). Oligonucleotides were fixed onto the membrane by crosslinking with UV light (254mm) for 5 minutes. The membranes were then incubated for 1 hour at ambient temperature with a blocking solution of 10% fat-free dried skimmed milk in PBS solution. DNP-label detection was carried out by incubation for 1 hour at ambient temperature with either a 1 in 250 dilution of anti-DNP-horseradish peroxidase conjugate in PBS or a 1 in 500 dilution of anti-DNP-alkaline phosphatase conjugate in PBS solution. Both antibody-enzyme conjugates were supplied by Dakopatts. A coloured precipitate was obtained by either washing the membrane with a diaminobenzidine/hydrogen peroxide (DAB/H₂O₂) solution to produce a brown colour with horseradish peroxidase or with 5-bromo-4-chloro-3-indoyl phosphate/ nitroblue tetrazolium (BCIP/NBT) solution to give a dark blue colour with alkaline phosphatase. Both these solutions were prepared from tablets supplied by Sigma.

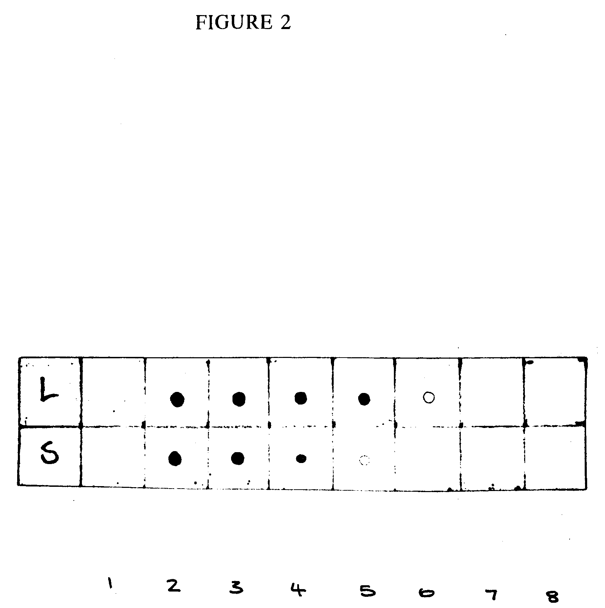

It is clear (figure 2) that the detection limit of an oligonucleotide bearing DNP groups attached by the C6 linker is significantly greater than that of the oligonucleotide without the linker.

### Example 4

### The effect of the number of DNP labels on oligonucleotide detection

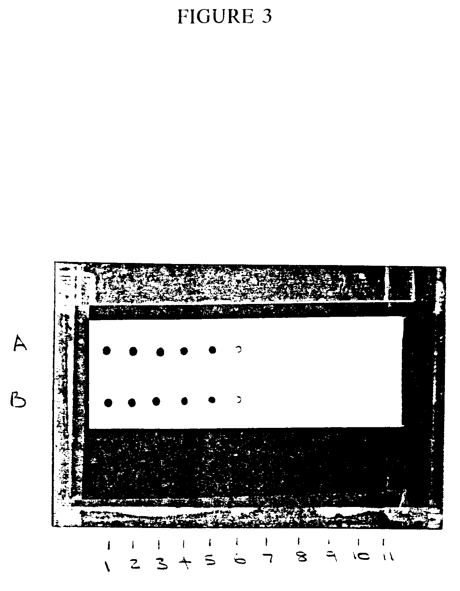

It has previously been shown that the sensitivity of detection of oligonucleotides with three terminal DNP groups is greater than for those with a single DNP group (see Brown et al, Nucleic Acid Research 21, No 8, p1705-1712 (1993)). The availability of an internal and a terminal DNP label now enabled us to investigate the effect of greatly increasing the number and altering the distribution of DNP labels in an oligonucleotide. Two oligonucleotide pools were prepared, each containing five oligonucleotide probes for M.a. paratuberculosis (see Stevenson K. et al., Biomedical Peptides, Proteins & Nucleic Acids (1994) 1, 17-20). The oligonucleotides in the first pool each contained 6 DNP groups, 3 terminal and 3 internal, and those in the second pool each contained 20 DNP groups, 3 terminal and 17 internal. A series of dilutions were made and aliquots were applied to a nylon membrane. In this experiment the membrane was developed using an anti-DNP/alkaline phosphatase conjugate and BCIP/NBT (see Cubie H. A. et al., J. Virol Methods (1995) 53 : 91-102). The results (figure 3) show that there is no advantage gained by incorporating large numbers of DNP labels in short oligonucleotides. For both DNP-labelled oligonucleotide pools detection of 320fg of total DNA was possible. The observed superiority of the antibody-alkaline phosphatase conjugate/BCIP/NBT system over the horseradish peroxidase/DAB system was consistent with other experiments carred out (see Cubie H. A. et al., in Mol. Cell Probes (1995) 9 : 59-66).

### Example 5

### Hybridisation of DNP-labelled oligonucleotides to immobilised target DNA

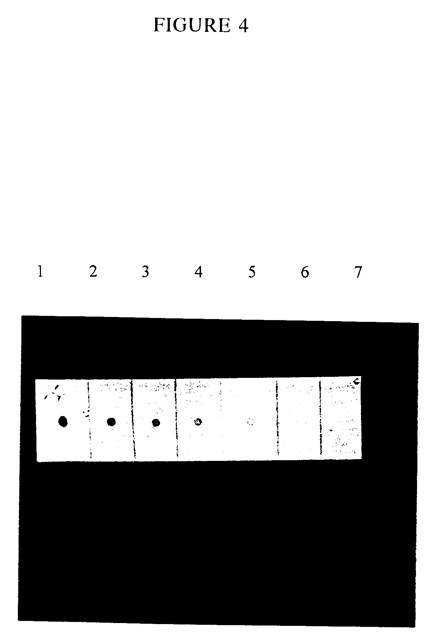

The hybridization properties of DNP labelled oligonucleotide probes were examined in the following way. A series of dilutions of double stranded DNA were made and samples were heated to 90°C, snap-cooled and applied to a nylon membrane. (The target DNA sequence from M.a. paratuberculosis was supplied by the Moredun Research Institute). The membrane was fixed, blocked and incubated for 1 hour at ambient temperature with 1000 fold excess of the above DNP labelled probe pool (5 oligonucleotides). The membrane was then developed using the anti-DNP/alkaline phosphatase conjugate and BCIP/NBT. The results (Figure 4) show that detection down to 0.2ng of target DNA was observed. No non-specific binding of the DNP-labelled probes to the non-target control DNA was observed.

### Example 6

### The use of DNP C6 dU in PCR primers

Hydrophobic reporter groups can adversely affect the performance of PCR primers, see Brown T. et al., "The use of 5'-chemically labelled PCR Primers" in PCR, essential data, ed. Newton, C.R., (1995) Bio Scientific Publishers (in press). To test whether oligonucleotides which are chemically labelled internally with DNP C6 dU can act as functional PCR primers the following three pairs of primers were synthesised:
C(+) 5'-GTT CGG GGC CGT CGC TTA GG
   C(-) 5'-CCC ACG TGA CCT CGC CTC CA
1(+) 5'-GTT CGG GGC CGT CGC TXA GG
   1(-) 5'-CCC ACG TGA CCT CGC CXC CA
3(+) 5'-GXT CGG GGC CGX CGC TXA GG
   3(-) 5'-CCC ACG XGA CCX CGC CXC CA

X=DNP C6 dU monomer.

All PCR experiments were carried out on a Techne PHC-3 thermal cycler using Taq polymerase and deoxynucleotide triphosphates purchased from Boehringer Mannheim. Primers were supplied by Oswel Research Products and the M.a paratuberculosis template was supplied by the Moredun Research Institute. The following PCR cycle was used and 30 cycles of PCR were carried out (steps 2 to 4).

Step 1, 94°C for 5 minutes; step 2, 94°C for 30 seconds; step 3, 55°C for 30 seconds; step 4, 72°C for 1 minute; step 5, 72°C for 2 minutes, step 6, hold at 4°C.

Each reaction tube contained 5µl of PCR x 10 buffer (Boehringer), 0.5µl dNTP mix (0.1mM in each of dATP, dCTP, dTTP and dGTP), Taq polymerase (2 Units), M.a. paratuberculosis DNA template (25ng) and primers (1µM each) made up to 5µl with distilled water and overlaid with 100µl of mineral oil. The PCR products were purified using the Wizard^{TM} DNA Clean-up system (Promega) following the manufacturers' instructions to remove primers, primer-dimers, enzymes and unincorporated triphosphates. Purified PCR products were analysed by agarose-gel electrophoresis with a 1% agarose gel containing 1µg/ml ethidium bromide in TBE buffer.

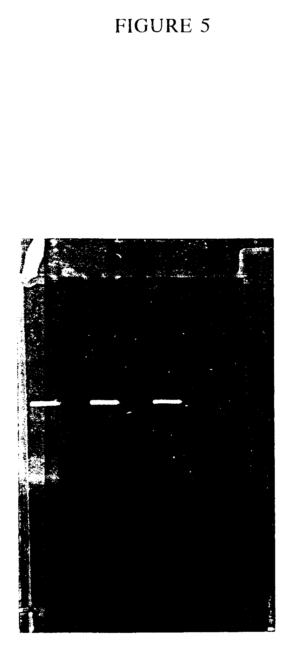

PCR reactions were carried out using each pair of primers and the results (figure 5) show that Taq polymerase is not inhibited by the presence of internal DNP labels in the primers. There is no significant difference in the amount of PCR product in the three experiments.

### Example 7

### Enzymatic Incorporation of DNP labels

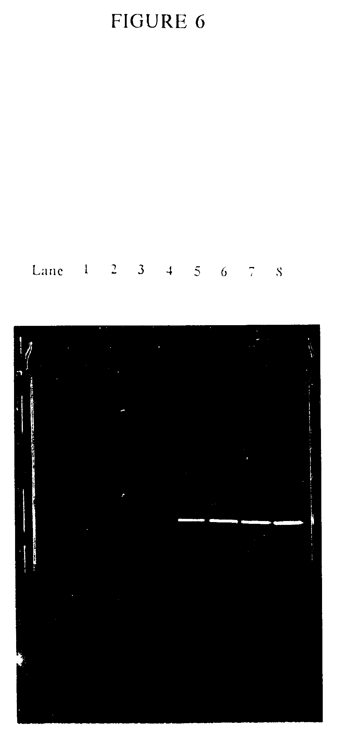

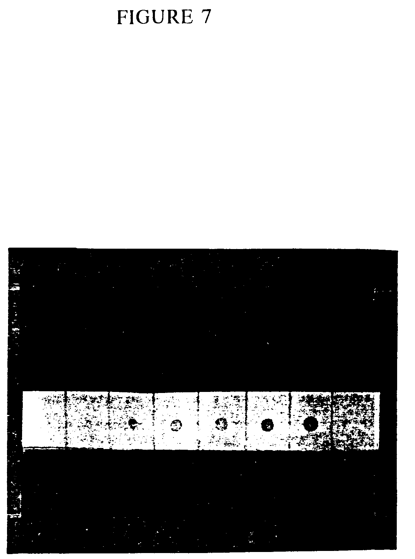

Chemically labelled deoxynucleoside triphosphates are poor substrates for DNA polymerase enzymes. To evaluate the enzymic incorporation of DNP C6 dU into nucleic acid the triphosphate (Compound 11 of Example 1) was synthesized from (Compound 8 of Example 1) using the method described by Kovács T. and Ötvos L. Tetrahedron Letts. (1988) 36 : 4525-4528. It was then used as a substrate for Taq polymerase to incorporate DNP-labels into a 389 base pair double stranded PCR product. The results (Figure 6) show the expected decrease in product yield as the ratio of DNP C6 dUTP to TTP is increased. Aliquots of all eight purified PCR products were heated to 90°C, snap-cooled and spotted onto a nylon membrane. The membrane was developed using an anti-DNP-HRP conjugate and DAB (Figure 7). A positive colour reaction was obtained for products which could not be visualised by ethidium bromide staining and the optimum DNP C6 dUTP to TTP ratio for antibody mediated detection was found to be 1 to 2. This ratio strikes the best balance between PCR product yield and label incorporation and it is clear from this experiment that (Compound 11 of Example 1) is a viable alternative to digoxigenin and biotin linked dUTP derivatives.

### Example 8

### Ultraviolet melting experiments (Table 1)

It is important to verify that the DNP labelling groups do not inhibit duplex formation. Having shown that DNP-labelled oligonucleotides are able to hybridise specifically to complementary DNA strands on a nylon membrane and during *in situ* hybridisation we undertook to measure the melting temperatures of various DNP-labelled and unlabelled duplexes.

Ultraviolet melting temperatures (Tm) were determined at 260nm using a Perkin-Elmer Lambda 15UV spectrometer equipped with a Peltier block and controlled by an IBM PS2 computer. A heating rate of 0.9K per minute was used throughout and the crude data was processed using the PECSS2 software package. The oligonucleotides were dissolved in a buffer consisting of aqueous sodium chloride (1.0M), sodium dihydrogen orthophosphate (0.01M), disodium EDTA (1mM) adjusted to pH 7.0. All experiments were repeated in triplicate and further experiments were carried out if the variation was greater than 0.5°C. The concentrations of the duplexes used were calculated to provide an absorbance reading of 1.55 at 80°C (see Table 1).

In general the results demonstrate that DNP C6 dU and DNP glycerol units have a stabilising effect on DNA duplexes. For example, the presence of one DNP C6 dU label in a deoxyoctamer duplex caused an increase of 4°C in melting temperature relative to the native duplex (exp 1, exp 2 and exp 3). It is well known that an overhanging single strand of DNA has a stabilising effect on the DNA duplex (exp 8, exp 9) but the effect of combining three glyceryl-based DNP labels at the 5'-end of the deoxyoctamer duplex and one internal DNP group is even greater (exp 4, exp 5) producing a further increase in of 4°C in T_{M}. When terminal DNP glycerol residues are opposed by normal DNA bases in the complementary strand a very stable duplex is obtained unlike duplexes with terminal mismatched base pair (exp 10, exp 11, exp 12, exp 13).

If both strands have overhanging 5'-DNP glycerol monomers (exp 1, exp 7) very large increases in DNA duplex stability are observed (ΔT_{M}=18°C). Stable duplexes are also obtained if both strands contain a DNP C6 dU residue (exp 6). Although neither is a relevant model for immunoblotting or *in situ* hybridisation experiments where only one strand is labelled with DNP groups, the increase in duplex stability caused by the DNP groups is interesting.

In contrast to DNA duplexes, studies on DNA/RNA hybrids revealed no increase in melting temperature due to incorporation of internal DNP C6 dU units (exp 14, exp 19). In general deoxyoligonucleotides containing DNP C6 dU formed less stable hybrids with RNA than with DNA (Compare exp 15 and exp 16 with exp 17).

Further UV melting experiments were carried out using a series of oligonucleotides and the results are summarised in Tables 2 and 3.

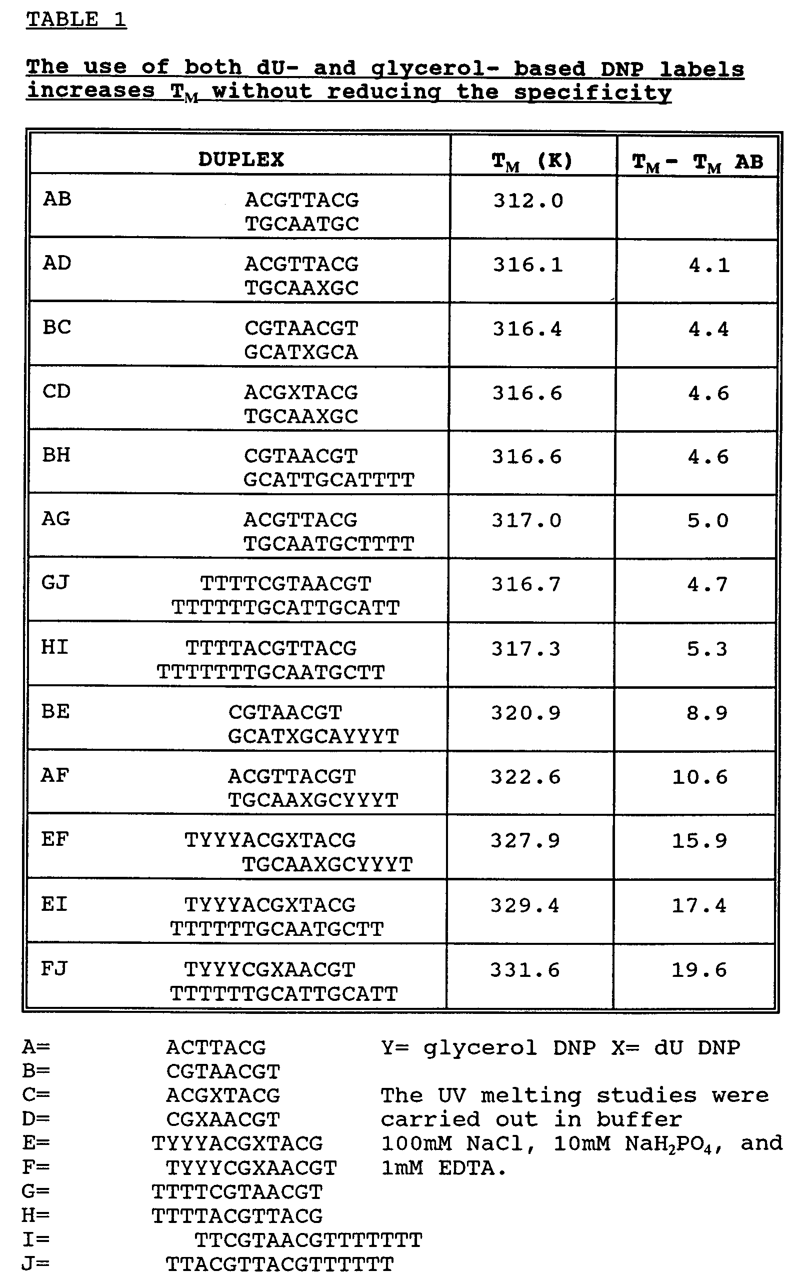

| Oligonucleotides Used in Further UV Melting Experiments | | | | | |
|---|---|---|---|---|---|
| | 5' | | | | 3' |
| a | ACA | TAC | TTG | ATC | |
| b | GAT | CAA | GTA | TGT | |
| c | ACA | XAC | TTG | ATC | |
| d | GAT | CAA | GXA | TGT | |
| e | ACA | XAC | XTG | ATC | |
| f | GAT | CAA | GXA | XGT | |
| g | ACA | XAC | XTG | AXC | |
| h | GAX | CAA | GXA | XGT | |
| i | TTT | ACA | TAC | TTC | ATC |
| j | TTT | GAT | CAA | GTA | TGT |
| k | YYY | ACA | TAC | TTG | ATC |
| l | YYY | GAT | CAA | GTA | TGT |
| m | YYY | ACA | XAC | TTG | ATC |
| n | YYY | GAT | CAA | GXA | TGT |
| o | YYY | ACA | XAC | XTG | ATC |
| p | YYY | GAT | CAA | GXA | XGT |
| q | ACA | UAC | UUG | AUC | |
| r | GAU | CAA | GUA | UGU | |
| s | UUU | ACA | UAC | UUG | AUC |
| t | UUU | GAU | CAA | GUA | UGU |
| X = dUDNP Y = glyceryl DNP | | | | | |

**TABLE 2**

| Experiment | T_{M}/°K | ΔT_{M}/°K |
|---|---|---|
| ab (Control) | 323.04 | - |
| ad | 325.68 | 2.64 |
| bc | 324.77 | 1.73 |
| af | 325.49 | 2.45 |
| be | 325.76 | 2.72 |
| ah | 325.82 | 2.78 |
| bg | 326.41 | 3.37 |
| aj | 326.06 | 3.02 |
| bi | 326.01 | 2.97 |
| al | 328.32 | 5.28 |
| bk | 327.36 | 4.32 |
| an | 329.69 | 6.65 |
| bm | 328.20 | 5.16 |
| ap | 330.30 | 7.26 |
| bo | 329.55 | 6.51 |
| qr (RNA duplex) | 333.52 | 10.48 |
| qt (RNA duplex) | 334.27 | 11.23 |
| rs (RNA duplex) | 333.97 | 10.93 |
| ar (RNA/DNA duplex) | 320.13 | -2.91 |
| bq (RNA/DNA duplex) | 318.45 | -4.59 |
| at (RNA/DNA duplex) | 322.56 | -0.98 |
| bs (RNA/DNA duplex) | 320.04 | -3.00 |
| cr (RNA/DNA duplex) + 1 DNP | 318.23 | -4.81 |
| dq (RNA/DNA duplex) + 1 DNP | 317.69 | -5.39 |
| ct (RNA/DNA duplex) + 1 DNP | 320.03 | -3.01 |
| ds (RNA/DNA duplex) + 1 DNP | 319.09 | -3.95 |

**TABLE 3**

| Summary | | |
|---|---|---|
| Effect studied | Expt. | Average ΔTm (compared to control) |
| duDNP in DNA duplex | ad, bc | +2.2°C |
| duDNP in DNA duplex | af, be | +2.6°C |
| duDNP in DNA duplex | ah, bg | +3.1°C |
| overhang | aj, bi | +3.0°C |
| glyceryl DNP in DNA duplex | al, bk | +4.8°C |
| glyceryl DNP in DNA duplex + 1 duDNP | an, bm | +5.9°C |
| glyceryl DNP in DNA duplex + 2 duDNP | ap, bo | +6.9°C |
| RNA duplex | qr | +10.5°C |
| RNA duplex with 3U overhang | qt, rs | +11.1°C |
| RNA/DNA hybrid | ar, bq | -3.8°C |
| RNA/DNA hybrid + 3U overhang | at, bs | -2.0°C |
| RNA/DNA hybrid + 1 duDNP | cr, dq | -5.1°C |
| RNA/DNA hyrbid + 1 duDNP + 3U overhang | ct, ds | -3.5°C |

## Claims

1. A compound of formula I:
X-Q-R*-NH-DNP (I)
wherein
DNP is a 2,4-dinitrophenyl group;
R* is a C1-12 carbon chain, optionally interrupted with one or more of the following: -0-, -NH-, alkylamine, carbon-carbon double bond, carbon-carbon triple bond, carbonyl group or amide group;
Q is a group Q* or a group 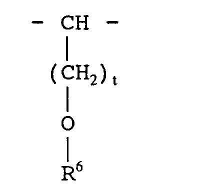
wherein t is 0 or is an integer of from 1 to 6;
Q* is a nucleoside comprising a heterocyclic amine base bonded to C1' of a C₅ or C₆ pentose sugar;
X is a group 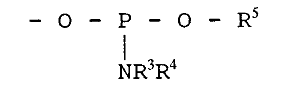
and where group Q represents a group Q*, X may also be a group 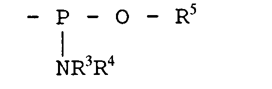
or a phosphate, diphosphate or triphosphate group;
R³ and R⁴ are each independently selected from C₁₋₆ alkyl, cycloalkyl, aralkyl and aryl groups; and
R⁵ and R⁶ are each blocking groups which may be the same or different.

2. A compound as claimed in Claim 1 wherein X is a group 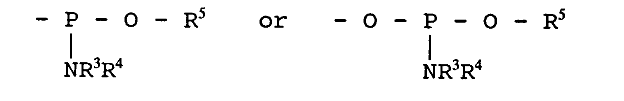 wherein groups R³, R⁴ and R⁵ are as defined in Claim 1.

3. A compound as claimed in Claim 1 wherein X is a triphosphate group.

4. A compound as claimed in any one of Claims 1 to 3 wherein R⁵ and R⁶ are each independently 2-cyanoethyl or p-dimethoxytrityl.

5. A compound as claimed in Claim 4 wherein group R⁵ is 2-cyanoethyl and group R⁶ is p-dimethoxytrityl.

6. A compound as claimed in any one of Claims 1 to 5 wherein group R* is a group (CH₂)ₙ-0-(CH₂)ₘ, -C=CR¹-, -C≡C- or -C-CR¹R², wherein R¹ and R² may each independently be a hydrogen atom, C₁₋₆ alkyl, a group -(CH₂)ₚNHC=O(CH₂)_{q}-, wherein n, m, p and q may each independently be 0 or an integer of from 1 to 12.

7. A compound as claimed in Claim 6 wherein group R* is a methylene, ethylene, n-propylene or a n-butylene group, or a group -(CH₂)ᵣ-NHC(=O)-(CH₂)ₛ-, wherein r is 1, 2 or 3 and s is 3, 4, 5 or 6.

8. A compound as claimed in any one of Claims 1 to 7 wherein group Q* is a purine, pyrimidine, alkylated purine or alkylated pyrimidine group.

9. A compound as claimed in Claim 8 wherein group Q* is uracil or methylated uracil.

10. A compound as claimed in Claim 1 of general formula IIa to IId 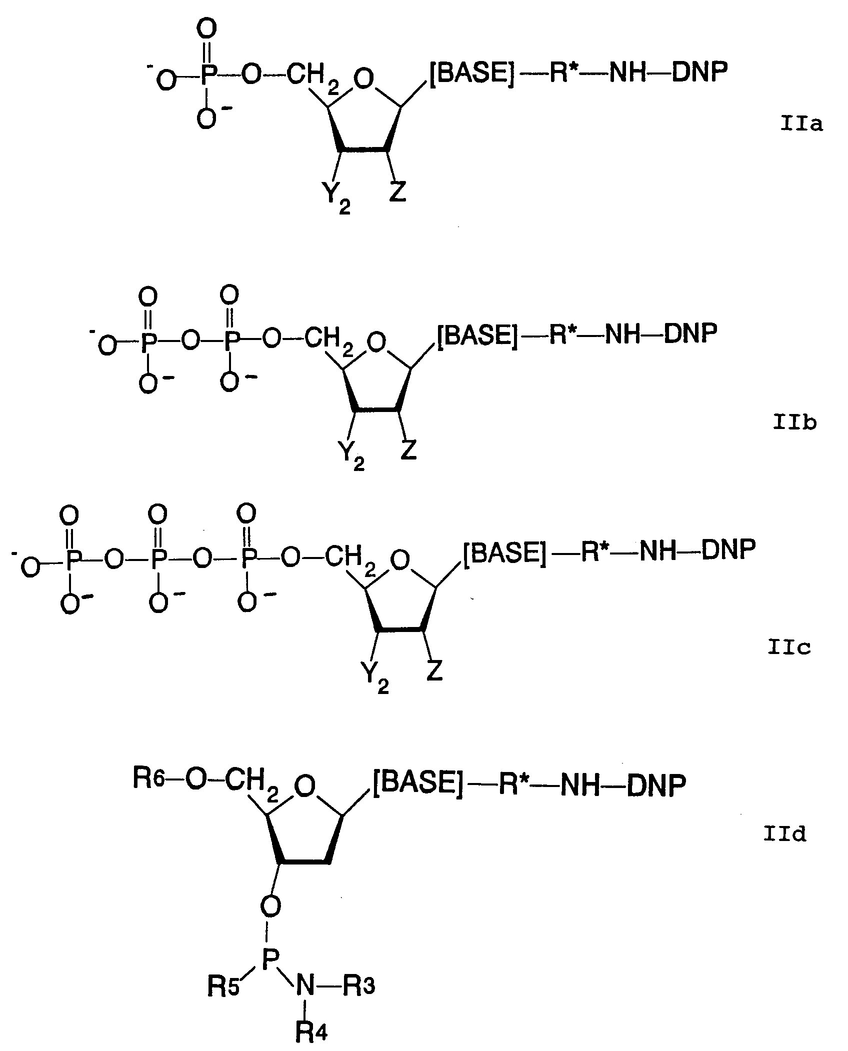
wherein groups R*, R³, R⁴, R⁵ and R⁶ are as defined in Claim 1;
[BASE] refers to a heterocyclic amine base (for example a pyrimidine or purine, or an alkylated derivative thereof;
Group Y₂ is a hydrogen atom or a hydroxyl group; and group Z represents a hydrogen atom, or a group -OR⁷, -NHR⁷, -SR⁷, where R⁷ is a blocking group, a C₁₋₆ alkyl group or a hydrogen atom.

11. A compound as claimed in any one of Claims 1 to 9 wherein group Q* is of general formula IIe 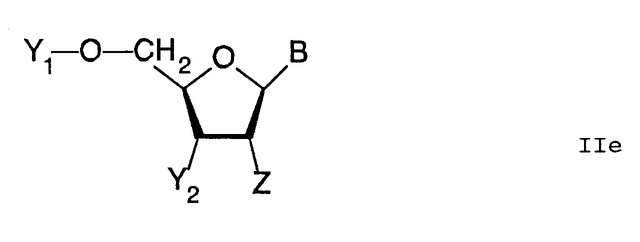 wherein:
one of groups Y₁ and Y₂ represents a group X and the other of groups Y₁ and Y₂ represents a hydrogen atom, or an hydroxyl, C₁₋₆ alkyl or -OR⁶ group;
group Z represents a hydrogen atom, or a group -OR⁷, -NHR⁷, -SR⁷ where R⁷ is a blocking group, a C₁₋₆ alkyl group or a hydrogen atom;
R⁶ is as defined above; and
B represents a covalent bond linking the pentose sugar moiety to the heterocyclic amine base of group Q*.

12. A compound as claimed in Claim 11 wherein group Y, is a mono-, di- or tri-phosphate group and group Y₂ is a hydrogen atom or a hydroxyl group.

13. A compound as claimed in Claim 11 wherein group Y₂ is a group 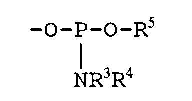 wherein groups R³, R⁴ and R⁵ are as defined in Claim 1, and group Y₁ is a group OR⁶, wherein group R⁶ is as defined in Claim 1.

14. A compound as claimed in any one of Claims 11 to 13 wherein the pentose sugar moity is 2-deoxyribose, 2,3-dideoxyribose or 2'-O-alkylribose.

15. A compound as claimed in Claim 1 of formula IIIa or IIIb.

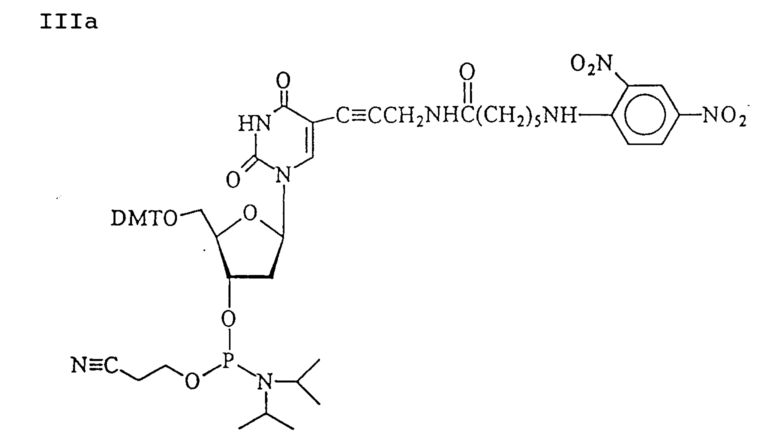

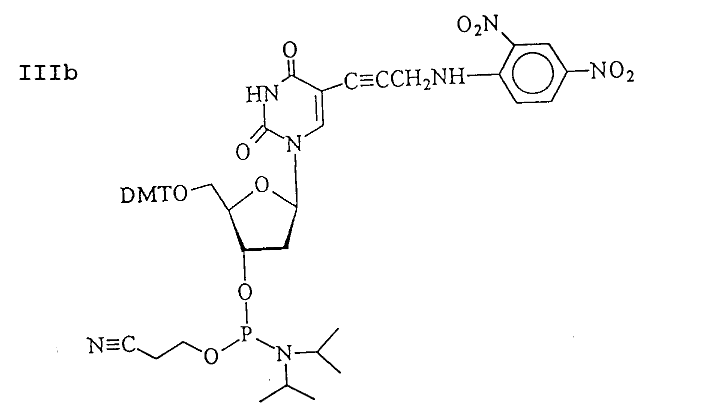

16. The use of a compound as claimed in any one of Claims 1 to 15 in the synthesis of oligonuceotide probes.

17. The use of a DNP-labelled oligonucleotide formed using a compound as claimed in any one of Claims 1 to 15 for solid phase PCR, solid phase in situ hydridisation or in affinity chromatography.

18. A process for labelling a hydroxyl group containing chemical moiety, said process comprising providing a compound as claimed in any one of Claims 1 to 15 and contacting said compound with said hydroxyl group containing moiety for a period of time sufficient for the labelling reaction to occur, and optionally detecting and/or purifying said labelled chemical moiety.
